# EUROPÄISCHE PATENTSCHRIFT

(11) **EP 0 905 515 B1**
(45) Veröffentlichungstag und Bekanntmachung des Hinweises auf die Patenterteilung: **08.12.2004**
(21) Anmeldenummer: 98118034.2
(22) Anmeldetag: 23.09.1998
(51) Int. Cl.: G01N 33/52, G01N 33/543, G01N 33/551

(54) **Analytisches Messverfahren und seine Verwendung**
Analytical measuring method and its use
Méthode pour la mésure analytique et son usage

(30) Priorität: 25.09.1997 DE 19742246
(43) Veröffentlichungstag der Anmeldung: 31.03.1999
(73) Patentinhaber: BASF AKTIENGESELLSCHAFT, 67056 Ludwigshafen (DE)
(72) Erfinder: Eipel, Heinz, 64625 Bensheim (DE); Keller, Harald, Dr., 67069 Ludwigshafen (DE); Kröger, Burkhard, Dr., 67117 Limburgerhof (DE); Philipp, Sabine, Dr., 64546 Mörfelden-Walldorf (DE)

(56) Entgegenhaltungen:
- EP-A- 0 895 082
- WO-A-93/22678
- WO-A-95/35505
- WO-A-97/40383
- WO-A-97/45730
- WO-A-98/01533
- DE-A- 19 628 928
- US-A- 5 424 219
- US-A- 5 653 939

## Beschreibung

Die Erfindung betrifft ein analytisches Meßverfahren.

Weiterhin betrifft die Erfindung die Verwendung des analytischen Meßverfahrens in der Diagnostik, in der Wirkstoffsuchforschung, in der kombinatorischen Chemie, im Pflanzenschutz, in der Toxikologie oder im Umweltschutzbereich.

Eine Hauptaufgabe der Wirkstoffsuchforschung im Pflanzenschutz oder in der Medizin ist die Identifizierung neuer Leitstrukturen und die Entwicklung von Wirkstoffen, die aus diesen Strukturen hervorgehen.

In der klassischen Wirkstoffsuchforschung wurde die biologische Wirkung neuer Verbindungen in einem Zufalls-Screening am ganzen Organismus beispielsweise der Pflanze oder dem Mikroorganismus getestet. Dabei wurden komplexe In-vitro- und In-vivo-Testmethoden eingesetzt, mit denen pro Jahr nur einige hundert Substanzen getestet werden konnten.

Die biologische Testung war dabei gegenüber der Synthesechemie der limitierende Faktor.

Durch die Bereitstellung molekularer Testsysteme, die in der Molekular- und Zellbiologie entwickelt wurden, hat sich die Situation drastisch verändert. Diese molekularen Testsysteme wie beispielsweise Rezeptorbindungsassays, Enzymassays oder Zell-Zellinteraktionsassays lassen sich in der Regel gut in Mikrotiterplatten in Reaktionsvolumina zwischen 5 bis 250 µl durchführen und einfach automatisieren. Dabei werden Mikrotiterplatten mit 96, 384, 864 und neuerdings sogar mit 1536 Reaktionsgefäßen auf einem einzigen Träger verwendet. Die Automatisierung und Miniaturisierung dieser Testsysteme ermöglicht einen hohen Probendurchsatz. Durch diese Entwicklung lassen sich große Zahlen verschiedener Chemikalien für eine mögliche Verwendung als Leitstruktur in der Wirkstoffsuchforschung testen.

Ein modernes automatisiertes Testsystem ermöglicht in einem Massenscreening die Prüfung von 100.000 und mehr Chemikalien pro Jahr auf ihre biologische Wirkung. Mikrotiterplattenassays werden sehr häufig verwendet, da sie in der Regel sehr robust sind.

Ein Nachteil der vorhandenen Testsysteme beispielsweise in der Wirkstoffsuchforschung, in der Diagnostik, im Umweltschutz oder Pflanzenschutz ist, daß für viele Testsysteme die benötigten Reagenzien wie beispielsweise Enzyme, Antikörper, Rezeptoren, Fluoreszenzfarbstoffe, radioaktiv oder sonstig markierte Liganden, Cytokine, Aktivatoren, Inhibitoren oder sonstige Reagenzien teuer, schwer herstellbar sind und/oder nicht in ausreichender Menge für die automatisierten Tests zur Verfügung stehen. Dies führt zu erheblichen Kosten beim automatisierten Screening nach Wirkstoffen. Eine weitere Miniaturisierung der Testansätze ist daher erstrebenswert.

In der unter dem Aktenzeichen 196.28.928.9 angemeldeten deutschen Patentanmeldung wird ein fester Träger für analytische Meßverfahren beschrieben, der eine weitere Miniaturisierung der Reaktionsvolumina ermöglicht. Mit diesen Trägern kann die Oberflächenspannung, die einer weiteren Miniaturisierung der vorhandenen Mikrotiterplattentechnik zu immer kleineren Reaktionslöchern (= Wells) hin im Wege steht, da durch sie in sehr kleinen Mikrotiterplattenvertiefungen Kräfte wie die Adhäsion der Reaktionsflüssigkeit an die Oberfläche der Mikrotiterplatten oder die Kapillarkräfte eine immer größere Rolle spielen und so eine Befüllung der Reaktionslöcher und damit eine Messung unmöglich machen, vorteilhaft zur weiteren Verringerung der Reaktionsvolumina genutzt werden. In dieser Anmeldung wird ein analytisches Meßverfahren beansprucht, bei dem mit Hilfe eines Mikro-Dosiersystems (Firma Microdrop) die verschiedenen Reagenzien des Tests aufgebracht werden. Nachteil dieses Verfahrens ist, daß obwohl ein für alle Meßbereiche gleiches Reagenz benutzt wird, dieses in sequentiellen Arbeitsgängen auf die verschiedenen Meßbereiche mit Hilfe des Mikrodosiersystems aufgebracht werden muß.

WO 95/35505 offenbart die Verwendung eines Trägers, der hydrophile Meßbereiche aufweist, die von einem hydrophoben Gitter umgeben sind.

Es bestand daher die Aufgabe, ein einfaches, rasches und preiswertes analytisches Meßverfahren zu entwickeln und für die Wirkstoffsuchforschung, die Diagnostik, den Umweltschutz, den Pflanzenschutz, der Toxikologie oder für die kombinatorische Chemie zur Verfügung zu stellen.

Die gestellte Aufgabe wurde durch ein analytisches Meßverfahren unter Verwendung eines festen Trägers, der im wesentlichen aufgebaut ist aus einem inerten festen Trägermaterial, auf dem hydrophile Meßbereiche, die gegebenenfalls mit einer Oberflächenladung versehen sind, durch mindestens eine hydrophobe Beschichtung voneinander getrennt sind, wobei auf dem Träger größer oder gleich 10 Meßpunkte pro cm² aufgebracht sind, dadurch gekennzeichnet, daß man folgende Arbeitsschritte durchführt:
a) einmaliges oder mehrmaliges Aufbringen von einander verschiedener Reagenzien einzeln oder im Gemisch auf die einzelnen hydrophilen Meßbereiche des Trägers,
b) Behandlung des Trägers mit mindestens einem für alle hydrophilen Meßbereiche gemeinsamen Reagenz, so daß das Reagenz gleichzeitig auf mehrere oder alle der hydrophilen Meßbereiche gebracht wird,
c) ggf. gemeinsames Waschen aller Meßbereiche nach dem Aufbringen der Reagenzien oder nach Ablauf der Reaktionszeit der Reagenzien untereinander,
d) Abdecken des Meßbereiche nach dem Aufbringen der Reagenzien oder nach dem Waschen aller Meßbereiche mit einer hydrophoben Schicht zur Verhinderung der Verdunstung der Reaktionsflüssigkeit,
e) gemeinsame oder einzelne Messung der Meßbereiche, gelöst.

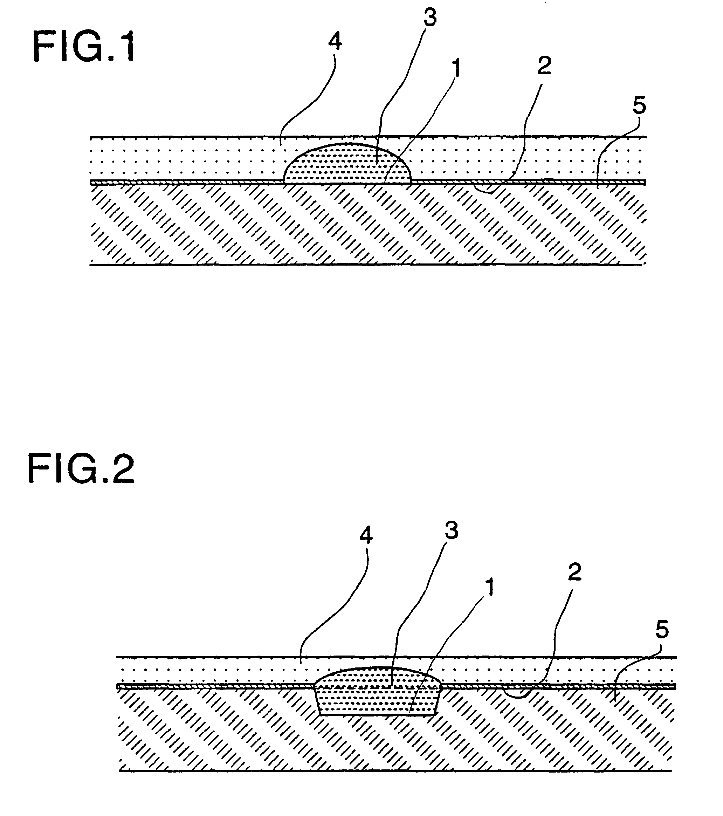

Unter dem festen Träger im erfindungsgemäßen Verfahren ist ein inerter fester Träger, der aus einer ebenen, planaren Platte eines eben solchen Blockes oder einer Folie beliebiger Form und Größe bestehen kann, in den gegebenenfalls kleine Mulden (siehe Figur 2) an den Stellen der Meßbereiche eingebracht sein können, zu verstehen. Bevorzugt werden plane Träger (siehe Figur 1). Bevorzugt sind rechteckige oder quadratische Träger, besonders bevorzugt sind rechteckige Träger in Größe einer Standardmikrotiterplatte (127,5 mm x 85,5 mm) oder ganzzahlige Vielfache der Mikrotiterplatten, die größer oder kleiner sein können wie beispielsweise die sogenannten Terasaki-Platten (81 mm x 56 mm, 60 Meßpunkte). Die bevorzugte Größe der erfindungsgemäßen Träger hat den Vorteil, daß die gesamte Peripherie der automatisierten Mikrotiterplattentechnik ohne Umbau verwendet werden kann. Eine weitere bevorzugte Ausführungsform des Trägers sind Träger in der Form handelsüblicher Objektträger für die Mikroskopie oder ganzzahliger vielfacher davon, da sie eine leichte, preiswerte Auswertung beispielsweise mit einem Mikroskop oder einem "Scanning-Mikroskop" und einem vorteilhafterweise automatisierten Auswertesystem ermöglichen.

Die Träger können beispielsweise aus Materialien bestehen wie Glas, Keramik, Quarz, Metall, Stein, Holz, Kunststoff, Gummi, Silicium, Germanium oder Porzellan. Die Materialien können in reiner Form, als Mischungen, Legierungen oder Blends oder in verschiedenen Schichten oder nach Beschichtung beispielsweise mit einem Kunststoff oder einem Lack zur Herstellung der erfindungsgemäßen Träger verwendet werden. Bevorzugt werden transparente Träger aus Quarz, Glas, Kunststoff, Germanium oder Silicium hergestellt, die für alle visuellen Tests wie mikroskopische, kameraunterstützte und/oder laserunterstützte Tests geeignet sind.

Als transparente Kunststoffe sind alle amorphen Kunststoffmaterialien, die einphasig oder mehrphasig mit gleichem Brechungsindex wie Polymere aus Acrylnitril-Butadienstyrol oder mehrphasig mit unterschiedlichem Brechungsindex, bei denen die Domänen der Kunststoffkomponenten Bereiche bilden, die kleiner als die Wellenlänge des Lichts sind wie beispielsweise die Blockcopolymere aus Polystyrol und Butadien (sog. Polystyrol/Butadienblends) geeignet.

Als besonders geeignete transparente Kunststoffe seien hier Polystyrol, Styrolacrylnitril, Polypropylen, Polycarbonat, PVC (= Polyvinylchlorid), Polymethylmethacrylat, Polyester, Silicone, Polyethylenacrylat, Polylactid oder Celluloseacetat, Cellulosepropionat, Cellulosebutyrat oder deren Mischungen genannt. Silicium- oder Germaniumträger eignen sich besonders für Anwendungen, bei denen eine Detektion oder Induktion der Reaktion über nahes Infrarotlicht erforderlich ist.

Es können auch Träger in Form eines Transportbandes verwendet werden, das bei Automatisierung der Assays an den Beschickungs-, Inkubations- oder Detektionsstationen vorbeilaufen kann.

Unter hydrophilen Meßbereichen des Trägers (5) sind Gebiete des Trägers zu verstehen, auf denen bzw. in denen nach Aufbringung der Reaktionsflüssigkeit und damit der Reagenzien bzw. Reaktanten die Messungen durchgeführt werden (siehe Nummer 1 in den Figuren 1 und 2). Sie entsprechen damit den "Wells" bzw. den Vertiefungen klassischer Mikrotiterplatten und werden nachstehend als "Meßbereiche oder Meßpunkte" bezeichnet.

Die hydrophilen Meßbereiche des Trägers sind vorteilhaft von einem hydrophoben Bereich (siehe Nummer 2 in den Figuren 1 und 2) umgeben. Dieser hydrophobe Bereich kann aus mindestens einer hydrophoben Beschichtung aufgebaut sein, die den Träger vollständig oder nur teilweise mit Unterbrechungen bedeckt.

Die Figuren 1 und 2 dienen der beispielhaften Verdeutlichung der Träger.

Die Meßbereiche sowie die sie voneinander trennenden hydrophoben Bereiche (siehe Nummer 2 in den Figuren 1 und 2) können beispielsweise durch Mikrolithographie-, Photoätz-, Mikrodruck- oder Mikrostempeltechnik aufgebracht werden oder mit Hilfe einer Maskentechnik aufgesprüht werden. Aus der Herstellungstechnik von Druckplatten sind photochemische Verfahren bekannt, mit denen Oberflächen von Platten oder Walzen gezielt an bestimmten Stellen hydrophob und an anderen Stellen hydrophil gemacht werden können.

Mit dieser Technik läßt sich beispielsweise auf einfache Weise auf einem Träger (5), z.B. auf einer Glas- oder Metallplatte, ein Raster von mehreren Tausend regelmäßig angeordneten hydrophilen Meßbereichen (siehe Nummer 1 in den Figuren 1 und 2), umgeben von hydrophoben Begrenzungen (siehe Nummer 2 in den Figuren 1 und 2), herstellen. Dabei können zunächst ein oder mehrere hydrophobe Beschichtungen auf den Träger aufgezogen werden und anschließend die Meßbereiche an den gewünschten Stellen aufgebracht werden oder umgekehrt zunächst die hydrophilen Meßbereiche und dann die hydrophoben Bereiche oder beides gleichzeitig aufgezogen werden. Es können auch mehrfach hydrophile Meßbereiche an die gleiche Stelle aufgetragen werden.

Bei hydrophoben Trägermaterialien werden sinnvollerweise hydrophile Meßpunkte auf dem Träger aufgebracht.

Die Meßbereiche können eine beliebige Form beispielsweise Punkte, Kreise, Polygone oder Kreuze haben, bevorzugt sind kreisförmige Meßbereiche.

Die hydrophilen Meßpunkte können vorteilhaft zur besseren Fixierung von Reagenzien noch weitere reaktive Gruppen tragen, die eine nicht-kovalente oder kovalente Bindung von Reagenzien ermöglichen. Bevorzugt werden Reagenzien, die eine kovalente Bindung ermöglichen, wie beispielsweise alle Kupplungsreagenzien aus der Peptid- oder Nucleinsäurechemie wie Aldehyd-, Epoxid-, Isothiocyanat-, Carbodiimid-, Hydroxyl-, Sulfhydryl-, Amino- und/oder Carboxylgruppen tragende Kupplungsreagenzien. Auch eine Bindung über Biotin/Avidin kann vorteilhaft genutzt werden.

Vorteilhafterweise werden, um eine möglichst hohe Belegungsdichte der Meßpunkte mit reaktiven Gruppen in den hydrophilen Meßpunkten zu erreichen, Polymere, die viele Bindungsstellen zur Verfügung stellen können, verwendet. Beispielhaft seien saure oder basische Gruppen tragende Polymere wie Polyimine, Proteine, Polylysin, Nukleinsäuren oder (Meth)acrylsäurecopolymere genannt. An diese Polymere können die Reagenzien direkt und/oder über bifunktionelle Kupplungsreagenzien gebunden werden.

Die hydrophobe Beschichtung oder Beschichtungen als separate Bereiche um die Meßbereiche liegen, bevorzugt sind hydrophobe Ringe, die die hydrophilen Meßbereiche voneinander trennen.

Prinzipiell ist es möglich, jede beliebige Anzahl von Meßpunkten auf einen Träger aufzubringen, bevorzugt sind größer oder gleich 10 Meßpunkte pro cm², besonders bevorzugt sind größer oder gleich 15 Meßpunkte pro cm², ganz besonders bevorzugt sind größer oder gleich 20 Meßpunkte pro cm². Am meisten bevorzugt werden Träger mit größer oder gleich 30 Meßpunkte pro cm². Dabei werden Reaktionsvolumina von wenigen nl bis zu einigen µl aufgetragen, bevorzugt werden Volumina kleiner 5 µl, besonders bevorzugt kleiner oder gleich 1 µl, aufgetragen.

Die Meßpunkte können in beliebigen Rastern auf den Träger aufgebracht sein, bevorzugt sind quadratische oder rechteckige Raster.

Zum Aufbringen von Probenmaterial und Reagenzien eignen sich alle Methoden, die Flüssigkeitsmengen zwischen wenigen nl und wenigen µl dosieren können, wie beispielsweise Techniken, die in Tintenstrahldruckern, sogenannte Ink-Jet-Technologie (siehe DE-A 40 24 544) oder in der Durchfluß-Zytometrie, in sogenannten Zellsorten (Horan, P.K., Wheeless, L.L., Quantitative Single Analysis and Sorting, Science 1977, 198, 149 - 157) verwendet werden. Die Tropfenbildung kann dabei mit piezoelektrischer Zertropfung (Ultraschall), piezoelektrischer Tropfenausschleuderung oder Ausschleuderung durch Verdampfung (Ink-Jet-Technik) erfolgen. Es können Systeme mit permanenter Tropfenerzeugung oder Systeme, die Tropfen auf Anforderung erzeugen, verwendet werden.

Mit diesen Techniken können einzelne Tröpfchen exakt dosiert und gezielt auf die einzelnen hydrophilen Meßpunkte der Multi-Analysen-Oberfläche des Trägers plaziert werden, indem zum Beispiel der Träger unter einer oder mehreren parallel angeordneten Düsen entsprechend dem Takt der dosierten Flüssigkeit und entsprechend dem vorgegebenen Raster bewegt wird. Ebenso kann auch die Dosiervorrichtung beispielsweise aus mindestens einer Düse über dem Träger entsprechend dem Takt der dosierten Flüssigkeit und entsprechend dem vorgegebenen Raster bewegt werden.

Mit diesen einzelne Tröpfchen aufbringenden Methoden lassen sich vorteilhafterweise einmalig oder mehrmalig von einander verschiedene Reagenzien einzeln oder im Gemisch auf die einzelnen hydrophilen Meßpunkte des Trägers aufbringen (Verfahrensschritt a).

Es zeigt sich, daß elektrostatische Aufladungen sowie Luftströmungen zwischen den Düsen und den Trägern - entgegen den Erwartungen - keinen Einfluß auf die exakte Plazierung der Reagenzien auf dem Träger haben. Im Gegenteil: diese technisch bedingten Ungenauigkeiten bei der Plazierung der Reagenzien werden durch eine automatische Nachfokussierung über hydrophile/hydrophobe Wechselwirkungen zwischen den vorteilhafterweise wässrigen oder polaren Reagenzflüssigkeiten und den hydrophoben und hydrophilen Bereichen des Trägers korrigiert. Bevorzugt werden wässrige Reagenzflüssigkeiten.

Darüber hinaus wurde gefunden, daß auch Reagenzien, die beispielsweise über Eintauchen oder Überspülen der gesamten Trägerfläche auf die Meßpunkte aufgetragen werden sich automatisch voneinander abgrenzen und auf die Meßbereiche fokussieren. Als weitere vorteilhafte gemeinsame Aufbringungsmethoden für die Reagenzien sei das Aufbringen mit einem Rakel oder mit einem Schwamm oder einem sonstigen saugfähigen Material genannt. Vorteilhaft können die Träger auch mit den Reagenzien besprüht werden. Mit dieser Methode läßt sich vorteilhafterweise mindestens ein gemeinsames Reagenz ein- oder mehrmalig gleichzeitig auf mehrere oder alle hydrophilen Meßpunkte des Trägers aufbringen (Verfahrensschritt b).

Eine zeitaufwendige sequenzielle und damit teure Aufbringung der Reagenzien, wie sie in der deutschen Anmeldung mit dem Aktenkennzeichen 196.28.928.9 beschrieben ist, ist damit nicht mehr erforderlich.

Auch die Waschschritte, die ggf. zwischen den einzelnen Verfahrensschritten (a) und/oder (b) oder vor der Messung (e) erforderlich sind können vorteilhaft durch Tauchen, Überspülen oder Überwischen mit einem saugfähigen Material durchgeführt werden. Diese Waschschritte aller Meßbereiche können nach dem Aufbringen der Reagenzien oder nach Ablauf der Reaktionszeit der Reagenzien untereinander erfolgen. Es können auch Waschschritte bei einem mehrmaligen Aufbringen von einander verschiedener Reagenzien auf die einzelnen hydrophilen Meßbereiche des Trägers zwischen den einzelnen Anwendungen durchgeführt werden oder aber ein gemeinsamer Waschschritt am Ende der Behandlungen durchgeführt werden.

Wurden ein oder mehrere Reaktionspartner an die hydrophilen Meßbereiche des Trägers gebunden, und werden anschließend gemeinsame Reagenzien durch die oben genannten Methoden aufgebracht, so kann die Reaktion sogar beispielsweise in einem Tauchbad durchgeführt werden, ohne daß die einzelnen Meßpunkte physikalisch voneinander getrennt sein müssen.

Selbst wenn sich geringe Mengen von freien Reaktanten in der Flüssigkeit befinden, so würde dies nur zu einer sehr geringen Kreuzkontamination führen, solange darauf geachtet wird, daß ein günstiges Verhältnis zwischen der eigentlichen Inkubationszeit und der Dauer des gemeinsamen Kontaktes aller Reaktanten und aller Meßpunkte eingehalten wird.

Die Verfahrensschritte (a) und (b) des erfindungsgemäßen analytischen Meßverfahrens können in beliebiger Reihenfolge ein- oder mehrmalig durchgeführt werden, wobei zwischen den Verfahrensschritten oder vor der oder den Messungen (e) gegebenenfalls überschüssige Reagenzien durch Waschen (c) und/oder Abstreifen entfernt werden.

Die von einander verschiedenen Reagenzien können einzeln oder in einem Gemisch auf die einzelnen hydrophilen Meßpunkte (= Meßbereiche) des Trägers aufgebracht werden.

Wie oben beschrieben, können mit diesem Meßverfahren verschiedene Reagenzien und/oder einzelne Zellen an die vorgegebenen Orte (= Meßpunkte) auf der Trägeroberfläche plaziert und zur Reaktion gebracht werden. Von Vorteil ist, daß bei den kleinen Volumina im Bereich von einigen Nanoliter bis zu wenigen Mikroliter eine Vermischung der Reaktanten durch Diffusion sehr rasch eintritt, so daß eine besondere mechanische Mischvorrichtung nicht notwendig ist. Es können auch vor der Zugabe von Flüssigkeitströpfchen zur Durchführung der eigentlichen Analyse gewisse Liganden, z. B. Proteine oder Nucleinsäuren, auf dem Träger in adsorbierter oder chemisch gebundener Form vor der Zudosierung der Meßproben und der Reagenzien bereits vorliegen.

Weitere Vorteile des erfindungsgemäßen Meßverfahrens sind die Einsparung an Substanzen wie beispielsweise an zu testenden Chemikalien, an Enzymen, an Zellen oder sonstigen Reaktanten, an Zeit durch eine weitere Steigerung gegebenenfalls automatisierter paralleler Reaktionsansätze, an Platz- und Personalbedarf, durch eine weitere Miniaturisierung der Reaktionsansätze.

Die auf den Trägern abgelegten Reagenztröpfchen können auch in Form von Geltröpfchen auf den Träger aufgebracht werden, die sich gegebenenfalls anschließend verfestigen und so die Verdunstung der Reaktionsflüssigkeit verringern.

Die Verdunstung der Reaktionsflüssigkeit (siehe Nummer 3 in den Figuren 1 und 2) wird durch Überschichtung mit einer hydrophoben Flüssigkeit (siehe Nummer 4 in den Figuren 1 und 2), wobei die hydrophobe Beschichtung oder Beschichtungen wie ein Anker wirken, verringert. Bevorzugt zur Überschichtung werden niedrigviskose Öle wie Silikonöle verwendet.

Die Verdunstung kann auch durch Inkubation der Träger in einer nahezu wasserdampfgesättigten Atmosphäre reduziert werden.

Durch Kühlung der Träger kann die Verdunstung ebenfalls reduziert werden.

Es können einzelne der genannten Elemente zur Verminderung der Verdunstung verwendet werden oder deren Kombinationen.

Im erfindungsgemäßen analytischen Meßverfahren kann je nach Aufgabenstellung und den verwendeten Reaktanten auch das zwischenzeitliche Eintrocknen der einzelnen Reaktionsansätze auf den hydrophilen Meßpunkten durchaus auch toleriert werden.

Das erfindungsgemäße analytische Meßverfahren eignet sich prinzipiell für alle heute in Mikrotiterplatten durchgeführten Analysenmethoden, wie beispielsweise kolorimetrische, fluorimetrische oder densitometrische Methoden. Dabei können die Lichtstreuung, Trübung, wellenlängenabhängige Lichtabsorption, Fluoreszenz, Lumineszenz, Raman-Streuung, ATR (= Attenuated Total Reflection), Radioaktivität, Isotopenmarkierung, pH-Veränderungen oder Ionenverschiebungen vorteilhaft alleine oder in Kombination genutzt und gemessen werden, um hier nur einige der möglichen Meßgrößen zu nennen.

Als mögliche im erfindungsgemäßen Meßverfahren durchführbare Analysenmethoden seien hier die Bindung von Antikörper an Antigene, die Wechselwirkung zwischen Rezeptoren und Liganden, die spezifische Spaltung von Substratmolekülen durch Enzyme, die Polymerase-Kettenreaktion ("PCR"), Agglutinationstests oder die Wechselwirkung zwischen verschiedenen oder gleichen Zelltypen wie Enzymassays, Titrationsassays wie Virustitrationsassays, Erythrozyten- oder Plättchenaggregationsassays, Agglutinationsassays mit Latexkügelchen, ELISA- (= Enzyme-linked immunosorbent assay) oder RIA- (= Radioimmunoassay) genannt.

Im erfindungsgemäßen analytischen Meßverfahren kann eine Messung der Reaktion mehrmalig zwischen den Verfahrensschritten oder Verfahrensteilschritten (= mehrmaliges Wiederholen eines Verfahrensschrittes) oder einmalig nach dem Aufbringen der Reagenzien und Ablauf der Reaktionszeit oder zwischen den Verfahrensschritten und nach dem Aufbringen der Reagenzien und Ablauf der Reaktionszeit erfolgen.

Das erfindungsgemäße analytische Meßverfahren kann beispielsweise in der Diagnostik, in der Wirkstoffsuchforschung, in der kombinatorischen Chemie, im Pflanzenschutz, in der Toxikologie, im Umweltschutz beispielsweise bei cytotoxikologischen Tests, in der Medizin oder der Biochemie eingesetzt werden.

Das erfindungsgemäße analytische Meßverfahren ist besonders für das Massenscreening geeignet.

Als Meßmethoden für das erfindungsgemäße Meßverfahren besonders geeignet sind alle modernen bilderfassenden und bildauswertenden Analysensysteme.

Die folgenden Beispiele dienen der weiteren Veranschaulichung der Erfindung, ohne sie in irgendeiner Weise einzuschränken.

### Beispiel 1

Herstellung eines Trägers aus einem Glasobjektträger zur Verwendung im erfindungsgemäßen Verfahren

Zunächst wurde der Glasobjektträger mit einer 20%igen wässrigen Lösung eines sauren Reinigers (Reacalc®, Firma Chemotec GmbH) in einem Ultraschalltauchbad 10 Minuten gereinigt. Anschließend wurde der Glasobjektträger mit Wasser und danach mit absolutem Ethanol gespült und bei ca. 23 °C getrocknet.

Mit einem Mikrostempel wurde auf dem hydrophilen Träger (5) die hydrophobe Beschichtung in Form von hydrophoben Ringen (siehe Figur 1 und 2) aufgebracht. Zur Aufbringung der hydrophoben Schicht wurde eine 1%ige Hexadecyl-trimethoxy-silanlösung in Isopropanol/H₂O (9:1) verwendet. Der in die Silanlösung getauchte Stempel wurde kurz - ca. 5 sec. - auf den Träger gedrückt und anschließend wurde der Träger 15 Minuten bei 100 °C getrocknet. Es wurden zwei Typen von Stempeln zum Aufbringen von 12 bzw. 25 Meßpunkten pro Quadratzentimeter verwendet.

## Patentansprüche

1. Analytisches Meßverfahren unter Verwendung eines festen Trägers, der im wesentlichen aufgebaut ist aus einem inerten festen Trägermaterial, auf dem hydrophile Meßbereiche, die gegebenenfalls mit einer Oberflächenladung versehen sind, durch mindestens eine hydrophobe Beschichtung in Form separater Bereiche um die hydrophilen Meßbereiche voneinander getrennt sind, wobei auf dem Träger größer oder gleich 10 Meßpunkte pro cm² aufgebracht sind, **dadurch gekennzeichnet, daß** man folgende Arbeitsschritte durchführt:
a) einmaliges oder mehrmaliges Aufbringen von einander verschiedener Reagenzien einzeln oder im Gemisch auf die einzelnen hydrophilen Meßbereiche des Trägers,
b) Behandlung des Trägers mit mindestens einem für alle hydrophilen Meßbereiche gemeinsamen Reagenz, so daß das Reagenz gleichzeitig auf mehrere oder alle der hydrophilen Meßbereiche gebracht wird,
c) ggf. gemeinsames Waschen aller Meßbereiche nach dem Aufbringen der Reagenzien oder nach Ablauf der Reaktionszeit der Reagenzien untereinander,
d) Abdecken der Meßbereiche nach dem Aufbringen der Reagenzien oder nach dem Waschen aller Meßbereiche mit einer hydrophoben Schicht zur Verhinderung der Verdunstung der Reaktionsflüssigkeit,
e) gemeinsame oder einzelne Messung der Meßbereiche.

2. Analytisches Meßverfahren nach Anspruch 1, **dadurch gekennzeichnet, daß** die Arbeitsschritte (a) und (b) des Verfahrens in beliebiger Reihenfolge ein- oder mehrmalig durchgeführt werden, wobei zwischen den Verfahrensschritten oder vor der Messung (e) gegebenenfalls überschüssige Reagenzien durch Waschen (c) entfernt werden.

3. Analytisches Meßverfahren nach Anspruch 1 oder 2, **dadurch gekennzeichnet, daß** die Messung (e) mehrmalig zwischen den Verfahrensschritten oder einmalig nach dem Aufbringen der Reagenzien und Ablauf der Reaktionszeit(en) oder zwischen den Verfahrensschritten und nach dem Aufbringen der Reagenzien und Ablauf der Reaktionszeit(en) erfolgt.

4. Analytisches Meßverfahren nach den Ansprüchen 1 bis 3, **dadurch gekennzeichnet, daß** man die Messung in nahezu wasserdampfgesättigter Atmosphäre durchführt.

5. Analytisches Meßverfahren nach den Ansprüchen 1 bis 4, **dadurch gekennzeichnet, daß** man die Messung unter Kühlung des Trägers durchführt.

6. Verwendung eines analytischen Meßverfahrens gemäß den Ansprüchen 1 bis 5 in der Diagnostik, in der Wirkstoffsuchforschung, in der kombinatorischen Chemie, im Pflanzenschutz, in der Toxikologie oder im Umweltschutzbereich.

## Claims

1. An analytical measurement method using a solid support which is essentially composed of an inert solid support material on which hydrophilic measurement zones which may be provided with a surface loading are separated from one another by at least one hydrophobic coating in the form of separate zones around the hydrophilic measurement zones, where the number of measurement points applied per cm² of the support is greater than or equal to 10, wherein the following steps are carried out:
a) application one or more times of mutually different reagents singly or in a mixture to the individual hydrophilic measurement zones on the support,
b) treatment of the support with at least one reagent common to all the hydrophilic measurement zones so that the reagent is placed simultaneously on a plurality or all of the hydrophilic measurement zones,
c) where appropriate washing all the measurement zones together after the application of the reagents or after the completion of the time for the reagents to react with one another,
d) covering the measurement zones after application of the reagents or after the washing of all measurement zones with a hydrophobic layer to prevent evaporation of the reaction liquid,
e) measurement, together or singly, of the measurement zones.

2. An analytical measurement method as claimed in claim 1, wherein steps (a) and (b) in the method are carried out in any sequence one or more times, where appropriate excess reagents being removed by washing (c) between the steps in the method or before the measurement (e).

3. An analytical measurement method as claimed in claim 1 or 2, wherein the measurement (e) takes place more than once between the steps in the method or once after the application of the reagents and completion of the time(s) for the reaction or between the steps in the method and after the application of the reagents and completion of the time(s) for the reaction.

4. An analytical measurement method as claimed in claims 1 to 3, wherein the measurement is carried out in an atmosphere which is virtually saturated with water vapor.

5. An analytical measurement method as claimed in claims 1 to 4, wherein the measurement is carried out while cooling the support.

6. The use of an analytical measurement method as claimed in claims 1 to 5 in diagnosis, in research looking for active substances, in combinatorial chemistry, in crop protection, in toxicology or in environmental protection.

## Revendications

1. Procédé de mesure analytique avec utilisation d'un support solide, qui est constitué pour l'essentiel d'un matériau support solide inerte, sur lequel des zones de mesure hydrophiles, qui sont éventuellement munies d'une charge superficielle, sont séparées les unes des autres par au moins une couche hydrophobe sous la forme de zones séparées autour des zones de mesure hydrophiles, tandis que sont disposés sur le support 10 points de mesure par cm² ou plus, **caractérisé par le fait qu'**on effectue les étapes opératoires suivantes:
a) dépôt en une ou plusieurs fois de réactifs différents les uns des autres, isolément ou en mélange sur les zones de mesure hydrophiles individuelles du support,
b) traitement du support avec au moins un réactif commun à toutes les zones de mesure hydrophiles, de telle sorte que le réactif soit appliqué simultanément sur plusieurs ou la totalité des zones de mesure hydrophiles,
c) éventuellement lavage commun de toutes les zones de mesure après dépôt des réactifs ou après la fin de la durée de réaction des réactifs entre eux,
d) revêtement des zones de mesure après dépôt des réactifs ou après le lavage de toutes les zones de mesure avec une couche hydrophobe pour empêcher l'évaporation du fluide de réaction,
e) mesure commune ou individuelle des zones de mesure.

2. Procédé de mesure analytique selon la revendication 1, **caractérisé par le fait que** les étapes opérationnelles (a) et (b) du procédé sont mises en oeuvre une ou plusieurs fois dans un ordre quelconque, tandis qu'entre les étapes opératoires ou avant la mesure (e) on sépare éventuellement les réactifs en excès par lavage (c).

3. Procédé de mesure analytique selon la revendication 1 ou 2, **caractérisé par le fait que** la mesure (e) s'effectue plusieurs fois entre les étapes opératoires ou une fois après le dépôt des réactifs et l'achèvement de la(des) durée(s) de réaction ou entre les étapes opératoires et après l'application des réactifs et le déroulement de la(des) durée(s) de réaction.

4. Procédé de mesure analytique selon les revendications 1 à 3, **caractérisé par le fait qu'**on effectue la mesure dans une atmosphère pratiquement saturée de vapeur d'eau.

5. Procédé de mesure analytique selon les revendications 1 à 4, **caractérisé par le fait qu'**on effectue la mesure tout en refroidissant le support.

6. Utilisation d'un procédé de mesure analytique selon les revendications 1 à 5 dans le diagnostic, dans la recherche de substances actives, dans la chimie combinatoire, dans la protection des plantes, dans la toxicologie ou dans le domaine de la protection de l'environnement.
